# EUROPEAN PATENT SPECIFICATION

(11) **EP 0 629 411 B1**
(45) Date of publication and mention of the grant of the patent: **31.10.2001**
(21) Application number: 94108880.9
(22) Date of filing: 09.06.1994
(51) Int. Cl.: A61L 15/18, A61L 15/60, C08K 3/36

(54) **Absorbent composition and disposable diaper containing the same**
Absorbierende Zusammensetzung und Wegwerfwindel mit dieser Zusammensetzung
Composition absorbante et couche à jeter contenant cette composition

(30) Priority: 18.06.1993 JP 17266993
(43) Date of publication of application: 21.12.1994
(73) Proprietor: Sanyo Chemical Industries, Ltd., Kyoto-shi Kyoto-fu, 605-0995 (JP)
(72) Inventor: Sumiya, Takashi, Fushimi-ku, Kyoto-shi 601-13 (JP); Date, Masashi, Moriguchi-shi, Osaka-fu, 570 (JP); Tanaka, Kenji, Otu-shi, Shiga-ken, 520-21 (JP)
(74) Representative: VOSSIUS & PARTNER

(56) References cited:
- EP-A- 0 471 595
- WO-A-90/08789
- WO-A-92/16565
- WO-A-93/17066
- GB-A- 2 082 614
- GB-A- 2 162 525

## Description

The present invention relates to a powdery or particulate absorbent composition which is suitably used for disposable diapers. More particularly, it relates to an absorbent composition having an excellent absorbing capacity as well as an excellent elasticity modulus after shearing of the gel and a moderate absorption rate, whereby excellent performance can be maintained for a long time. The invention also relates to the use of the absorbent composition in a disposable diaper and to disposable diapers comprising the said absorbent composition and a fibrous material.

Heretofore, powdery or particulate water swellable crosslinked polymers referred to as water absorbing resins have been used as absorbents for disposable diapers. Examples of the water absorbing resin include crosslinked copolymers of starch-acrylic acid salts, crosslinked polyacrylic acid salts, saponified products of crosslinked acrylic estervinyl acetate copolymers, crosslinked copolymers of maleic anhydride, and denaturated cellulose derivatives. Among them, crosslinked copolymers of starch-acrylic acid salts or crosslinked polyacrylic salts have been used for disposable diapers, heretofore. However, the amount of the water absorbing resin used in a conventional disposable diaper is small (e.g. about 15 to 25 % by weight) based on the total weight of fibrous material such as pulp and water absorbing resin.

As the disposable diaper became thin (increase in amount of the water absorbing resin, decrease in amount of pulp) recently, the ratio of the water absorbing resin to the fibrous material became large, and it became impossible to obtain satisfactory performance of the disposable diaper by employing a conventional water absorbing resin.

Namely, the absorbing capacity of the disposable diaper can be satisfied to some extent in a conventional disposable diaper, however, there is a problem that it is inferior in repeated absorbing capacity of urine because of low elasticity modulus after shearing of the absorbing gel, and diffusion property of urine is inferior and leakage is occuring because of unsuitable absorption rate. Furthermore, since the conventional disposable diaper is not designed for a thin type disposable diaper having a large ratio of the water absorbing resin to the fibrous material, balance between absorbency under load and elasticity modulus of the gel, shear-stability of the absorbing gel and diffusion property of urine are not actually taken into consideration. Particularly, performance of the disposable diaper depends largely on performance of the water absorbing resin in the disposable diaper wherein a ratio of the water absorbing resin to the fibrous material is large.

The requisite functions as the absorbent for the disposable diaper have been studied in the state at which the disposable diaper is actually used. As a result, it has been found that the following five functions are required:
(1) It absorbs urine sufficiently even in a state at which the user's weight is applied (absorbency under load);
(2) The absorbing gel is not deformed or broken due to the load (initial elasticity modulus);
(3) The absorbing gel is stable even if a shear force due to the user's locomotion (crawling, shifting of hip, turning-over, etc.) is applied, and it effects absorbing capacity to the next urination (elasticity modulus after shearing of the gel);
(4) No gel-blocking occurs on absorption of urine (decrease in content of fine powders); and
(5) It has a moderate absorption rate for diffusing urine widely through the disposable diaper (absorption rate, initial absorbency under load).

Namely, if these functions can be afforded to the absorbent, a disposable diaper having excellent dry feeling and no leakage after use for a long time can be realized. Particularly, as the ratio of the absorbent to the fibrous material becomes large (e.g. in the case that the weight ratio of the absorbent to the fibrous material exceeds 30/70), the above functions become necessary for the absorbent.

On the other hand, some suggestions of absorbents suitable for a disposable diaper were already made. For example, JP-A-62-54751 (U.S. Patent No. 4,654,039) suggests a non-grafted absorbent having a gel volume with respect to synthetic urine of at least 20 g/g, a shear modulus of the hydrogel of at least 2.10⁻⁴N/mm² (2,000 dyne/cm²) and an equilibrium extractable polymer content of not more than 17 % by weight. Some effect can be expected regarding items (2) and (3) among the above mentioned five functions because attention is paid only to the absorbing capacity under pressure-free state and the gel-strength as performances of the absorbent. However, it can not be said that the absorbent satisfies the other three functions sufficiently. Furthermore, JP-A-1-132802 (U.S. Patent No. 5,061,259) suggests a slightly-crosslinked absorbable gelatinizer having an equilibrium gel volume with respect to synthetic urine of at least 20 g/g and a mass median particle size of 400 to 1680 *µ*m. Some effect can be expected regarding item (4) among the above mentioned five functions because attention is paid only to the absorbing capacity under pressure-free state and the particle size as performances of the absorbent. However, it can not be said that the absorbable gelatinizer satisfies the other four functions sufficiently. Furthermore, JP-A-60-185804 and 60-185805 suggest an absorbent polymer having an absorbency of physiologic saline of 40 to 90 g/g, an absorption rate of 8 ml/0.3 g polymer or more and a gel-strength on saturation/swelling of deionized water of 33 to 200 g/cm². Some effect can be expected regarding the items (2) and (5) among the above mentioned five functions because attention is paid only to the absorbing capacity under pressure-free state, the absorption rate and the gel-strength to water as performances of the absorbent. However, it can not be said that the absorbent polymer satisfies the other three functions sufficiently.

It is an object of the present invention to provide an absorbent composition which is suitable for disposable diapers, particularly an absorbent composition for thin type disposable diapers wherein the absorbent is used in a high concentration or high amount. The absorbent composition should possess large absorbency under load with respect to urine and an excellent initial absorbency under load, whereby urine is sufficiently absorbed even in a state in which the user's weight is applied to the composition.

It is another object of the present invention to provide an absorbent composition having a large initial elasticity modulus, whereby the absorbing gel is not deformed or broken due to the user's weight in case of application for a disposable diaper.

It is still another object of the present invention to provide an absorbent composition having an excellent elasticity modulus after shearing of the urine absorbing gel, whereby it effects a stable absorbing capacity and it can absorb urine repeatedly even if a shear force is applied by the user's locomotion (e.g. crawling, shifting of hip, turning-over, etc.).

It is a further object of the present invention to provide an absorbent composition having a suitable absorption rate, whereby urine can be widely diffused throught the disposable diaper.

It is a still further object of the present invention to provide an absorbent composition having a good dry feeling of the gel after absorption of urine which results in non-slippery feeling.

It is another object of the present invention to provide an absorbent composition having a small content of fine powders and a narrow particle size distribution, whereby no gel-blocking occurs when it absorbs urine in case of application for disposable diaper, and urine can be widely diffused through the disposable diaper.

A further object of the present invention is to provide an absorbent composition wherein no dusting occurs and fluidity of powders is excellent, which results in excellent workability.

A still further object of the present invention is to provide an absorbent composition for disposable diaper, which does not escape from the fibrous material, whereby productivity during production of disposable diapers is improved (production loss is reduced).

These objects could be achieved on the basis of the finding that the objective absorbent can be obtained by mixing a hydrophilic silicon dioxide powder having a specific property into carboxylic group-containing water absorbing resin particles having a specific multi-crosslinked structure and further controlling a particle size distribution.

Namely, the present invention relates to a powdery or particulate absorbent composition suitable for disposable diapers which is used with a fibrous material, comprising a mixture of 100 parts by weight of water absorbing resin particles (A) and 0.05 to 5 parts by weight of a hydrophilic silicon dioxide fine powder (B), said composition having a particle size distribution such that the amount of particles having a particle size of larger than 850 *µ*m is not more than 10 % by weight and the amount of particles having a particle size of smaller than 150 *µ*m is not more than 10 % by weight;
wherein said water absorbing resin particles (A) comprise an acrylic acid salt and/or an acrylic acid as a main monomer of the resulting polymer, said water absorbing resin particleshaving a structure which is crosslinked with a first crosslinking agent (a) having at least two double bonds capable of copolymerizing with the monomer and a second crosslinking agent (b) having at least two functional groups capable of covalently binding to a carboxylic group; and wherein
said hydrophilic silicon dioxide fine powder (B) has a specific surface area of 50 to 450 m²/g and a water affinity of not less than 70 %.

The present invention furthermore relates to the use of the above mentioned absorbent composition in a disposable diaper.

In addition the present invention relates to a disposable diaper comprising the above mentioned absorbent composition and a fibrous material.

In the present invention, examples of the salt of acrylic acid include alkali metal salts (e.g. sodium salt, potassium salt and lithium salt), ammonium salts and amine salts (e.g. alkylamine salts with an alkyl group having 1 to 12 carbon atoms such as methylamine and trimethylamine salts; alkanolamine salts with an alkanol group having 2 to 12 carbon atoms such as triethanolamine and diethanolamine).

Among them, the preferable salts are alkali metal salts, and more preferable salts are sodium or potassium salts.

When using the mixture of an acrylic acid and an acrylic acid salt as the monomer, a molar ratio of the acrylic acid to the acrylic acid salt is normally (10 to 40) : (60 to 90), preferably (20 to 35) : (80 to 65). When the molar ratio of the acrylic acid is less than 10, the resulting water absorbing resin becomes alkaline. On the other hand, when it exceeds 40, the resulting water absorbing resin becomes acidic. Both cases are not preferred in view of safety to the skin of the human body and, therefore, these polymers are not suitable for a constituent component of the absorbent composition for disposable diaper.

When using the acrylic acid as the monomer, it is normal to partially neutralize carboxylic groups in the polymer with an alkaline substance after the completion of polymerization. Examples of the alkaline substance used for neutralization include alkali metal compounds (e.g. sodium hydroxide and potassium hydroxide), alkali metal carbonates (e.g. sodium carbonate and sodium bicarbonate), ammonia, amine compounds (e.g. alkylamines with an alkyl group having 1 to 12 carbon atoms such as methylamine and trimethylamine ; alkanolamines with an alkanol group having 2 to 12 carbon atoms such as triethanolamine and diethanolamine) and a mixture thereof. The neutralization degree is usually 60 to 90 mol %, preferably 65 to 80 mol %. When the neutralization degree is less than 60 mol %, the resulting water absorbing resin becomes acidic. On the other hand, when it exceeds 90 mol %, the resulting water absorbing resin becomes alkaline. Both cases are not preferred in view of safety to the skin of the human body and, therefore, these polymers are not suitable for a constituent component of the absorbent composition for disposable diaper.

In the present invention, examples of the first crosslinking agent (a) having at least two double bonds capable of copolymerizing with the monomer are as follows.
(1) Bis(meth)acrylamide:
   N,N' -alkylenebis(meth)acrylamides with an alkylene group having 1 to 6 carbon atoms such as N,N'-methylenebisacrylamide.
(2) Diester or polyester of polyols and unsaturated monocarboxylic or polycarboxylic acid:
   Di(meth)acrylic or tri(meth)acrylic acid esters of polyols [e.g. ethylene glycol, trimethylolpropane, glycerin, polyoxyethylene glycol and polyoxypropylene glycol], unsaturated polyesters [e.g. obtainable by reacting above mentioned polyols with unsaturated acids such as maleic acid] and di(meth)acrylic or tri(meth)acrylic acid esters [obtainable by reacting a polyepoxide with (meth)acrylic acid].
(3) Carbamylester:
   Carbamylesters obtained by reacting polyisocyanate [e.g. tolylene diisocyanate, hexamethylene diisocyanate, 4,4'-diphenylmethane diisocyanate or NCO group-containing prepolymer (obtainable by reacting the above mentioned polyisocyanate with a compound containing an active hydrogen atom)] with hydroxyethyl(meth)acrylate.
(4) Divinyl or polyvinyl compounds:
   E.g. divinylbenzene, divinyltoluene, divinylxylene, divinyl ether, divinylketone and trivinylbenzene.
(5) Di(meth)allyl or poly(meth)allyl ethers of polyols:
   Di(meth)allyl or poly(meth)allyl ether of polyols [e.g. alkylene glycol, glycerin, polyalkylene glycol, polyalkylene polyol and carbohydrates], e.g. polyethylene glycol diallyl ether, allylated starch and allylated cellulose.
(6) Diallyl or polyallyl ester of polycarboxylic acid:
   E.g. diallyl phthalate and diallyl adipate.
(7) Ester of unsaturated monocarboxylic or
   polycarboxylic acid and mono(meth)allyl ether of polyol:
   E.g. (Meth)acrylic acid esters of polyethylene glycol monoallyl ether.
(8) Polyallyloxyalkanes:
   E.g. diallyloxyethane, triallyloxyethane, tetraallyloxyethane, neopentyl glycol diallyl ether and pentaerythritol triallyl ether.

Among them, preferable crosslinking agents (a) are bis(meth)acrylamide (1), diester or polyester of polyols and unsaturated monocarboxylic or polycarboxylic acid (2), di(meth)allyl or poly(meth)allyl ethers of polyols (5) and polyallyloxyalkanes (8). More preferable crosslinking agents are N,N' -methylenebisacrylamide, ethylene glycol diacrylate, trimethylolpropane triacrylate, tetraallyloxyethane and pentaerythritol triallyl ether. Particularly preferable crosslinking agents are tetraallyloxyethane and pentaerythritol triallyl ether because they contain no functional group which is liable to be hydrolyzed (e.g. amide group or ester group) in the molecule.

The amount of the crosslinking agent (a) is normally 0.01 to 5 % by weight, preferably 0.05 to 3 % by weight, more preferably 0.1 to 1 % by weight, based on the total weight of the polymerizable monomers. When the amount of (a) is less than 0.01 % by weight, the resulting water absorbent resin is inferior in the elasticity modulus after shearing of the gel even if it is further crosslinked with a second crosslinking agent (b). On the other hand, when it exceeds 5 %, the initial elasticity modulus becomes too large to be brittle, which results in decrease of the elasticity modulus after shearing as well as absorbency under load.

In the present invention, the water absorbing resin is further crosslinked with a second crosslinking agent (b) having at least two functional groups capable of covalently binding to a carboxylic group. Examples of the crosslinking agent (b) include polyglycidyl ether compounds (e.g. ethylene glycol diglycidyl ether, glycerin-1,3-diglycidyl ether, glycerin triglycidyl ether and polyethylene glycol diglycidyl ether); polyol compounds (e.g. glycerin, ethylene glycol and polyethylene glycol); and polyamine compounds (e.g. ethylenediamine, diethylenetriamine, polyamide polyamine epichlorohydrin resin and polyamide epichlorohydrin resin). Among them, preferable crosslinking agents (b) are polyglycidyl ether compounds, polyol compounds and polyamine compounds.

Particularly preferable crosslinking agents (b) are ethylene glycol diglycidyl ether, glycerin-1,3-diglycidyl ether, polyamide polyamine epichlorohydrin resin and polyamine epichlorohydrin resin because they form a strong covalent bond together with the carboxylic group to give a water absorbing resin having an excellent elasticity modulus after shearing, and they can undergo a crosslinking reaction at a comparatively low temperature. Crosslinking agents which form an ionic bond together with the carboxylic group (e.g. zinc acetate, calcium acetate, barium acetate, strontium acetate, aluminum hydroxide, zirconium lactate and titanium lactate) are not included in the crosslinking agent (b) in the present invention. When the above ionizable polyvalent metal salts are used as the crosslinking agent (b), a positive ion is sometimes dissociated on absorption of urine and, therefore, good elasticity modulus after shearing of the gel can not be obtained.

The amount of the crosslinking agent (b) is normally 0.001 to 3 % by weight, preferably 0.005 to 2 % by weight, more preferably 0.01 to 1 % by weight, based on the total weight of the monomers. When the amount of (b) is less than 0.001 % by weight, the initial elasticity modulus of the resulting water absorbing resin becomes low and the elasticity modulus after shearing of the gel is also decreased. On the other hand, when it exceeds 3 %, the gel becomes too hard to be brittle, which results in decrease of the elasticity modulus after shearing as well as absorbency under load and absorption rate.

If necessary, the monomer of the acrylic acid salt and/or acrylic acid can be used in combination with other hydrophilic monomers. Examples of the hydrophilic monomer include unsaturated monocarboxylic or polycarboxylic acids [e.g. methacrylic acid, crotonic acid, maleic acid, itaconic acid and maleic anhydride]; monomers containing a sulfonic group [e.g. aliphatic or aromatic vinyl sulfonic acids such as vinyl sulfonic acid, allyl sulfonic acid, vinyl toluenesulfonic acid and styrene sulfonic acid, (meth)acryl sulfonic acids such as sulfoethyl (meth)acrylate and sulfopropyl (meth)acrylate, and (meth)acrylamide sulfonic acids such as 2-acrylamide-2-methylpropanesulfonic acid]; polymerizable monomers containing a phosphoric group [e.g. 2-hydroxyethyl (meth)acryloyl phosphate and phenyl-2-acryloyloxyethyl phosphate]; (meth)acrylamides and derivatives thereof; and vinyl acetate.

The amount of the other hydrophilic monomer is normally 0 to 30 % by weight, preferably 0 to 10 % by weight, based on the total weight of the monomers. When the amount of the other hydrophilic monomer exceeds 30 % by weight, the absorbency under load of the resulting water absorbing resin is reduced.

In the description herein, "(meth)acrylic-" means "acrylic-" or "methacrylic-".

As the polymerization method for producing the water absorbing resin, a known method may be employed, for example, a method of subjecting a polymerizable monomer and a crosslinking agent (a) to aqueous solution polymerization, a method of reverse phased suspension polymerization, a method of polymerizing by irradiation of radiation, electron ray or ultraviolet ray. Further,as the polymerization conditions (e.g. polymerization concentration, kind or amount of polymerization solvent and polymerization temperature), conventional known conditions may be employed.

In the present invention, it is an essential feature that the water absorbing resin is further crosslinked with a second crosslinking agent (b) having at least two functional groups capable of covalently binding with a carboxylic group after polymerization. This polymerization method is not limited to a specific one, and examples thereof include a method (1) comprising adding/kneading a crosslinking agent (b), which is optionally in the form of an aqueous solution, to a hydrogel polymer of the water absorbent resin, if necessary, partially neutralized with an alkali substance after polymerization and heat crosslinking (the hydrogel polymer may be dried and crosslinked, simultaneously); a method (2) comprising adding or spraying a crosslinking agent (b), which is optionally in the form of an aqueous solution, to the resulting powdered polymer obtained by drying/pulverizing the hydrogel polymer which is obtained by polymerization and is optionally adjusted to a desired particle size, and further crosslinking the polymer by a heat treatment; and a method (3) of using the crosslinking of the method (1) in combination with that of the method (2). The preferable methods are methods (1) and (3). By using these crosslinking methods, performances of the disposable diaper in case of application for disposable diaper becomes excellent.

The heat-crosslinking temperature of the hydrogel polymer or powdery or particulate mixture thereof which the crosslinking agent (b) has been added/kneaded is usually 80 to 220 °C, preferably 100 to 190 °C. When the temperature is less than 80 °C, it takes a long time to heat and is not economical and a crosslinking reaction sometimes does not proceed sufficiently to obtain the effect of the present invention by depending on the kinds or addition amount of (b). On the other hand, when the temperature exceeds 220 °C, coloring of the water absorbing resin and decrease in absorbency under load due to heat crosslinking may occur.

An apparatus for crosslinking may be any one which has hitherto been known. In the case of crosslinking according to the above method (1) or (2), as an apparatus for kneading the crosslinking agent (b) with the hydrogel polymer, for example there can be used a kneader, universal mixer, single or double-screw extruder or meat chopper. As an apparatus for heat crosslinking or drying, for example, there can be used a hot-air drier, drum drier, belt type drier or vacuum dryer.

In the case of surface crosslinking according to the method (2), as an apparatus for mixing with adding or spraying the crosslinking agent (b), for example, there can be used a screw blender, turbulizer, Nauta blender, V-shaped rotating mixer, ribbon mixer, double arm type kneader, fluidized bed mixer or air blender. As an apparatus for subjecting the mixture to a heat treatment, for example, there can be used a hot-air drier, airborne drier, fluidized bed drier, belt type drier, Nauta type heater, paddle drier or infrared drier.

The shape of the water absorbing resin particles (A) of the present invention may be any one which is powdery or particulate, for example, particle, granular, agglomerated, lamellar, lumpy or pearly shape.

The hydrophilic silicon dioxide fine powder (B) used in the present invention is dry silica produced by hydrolyzing silicon tetrachloride in a flame obtained by high temperature combustion of oxygen and hydrogen, which is normally referred to as "Fumed Silica". Accordingly, the hydrophilic silicon dioxide fine power (B) used in the present invention is normally non-porous silica.

Concretely, it is a hydrophilic silicon dioxide having a specific surface area of 50 to 450 m²/g and a water affinity of not less than 70 %. Preferably, it is a hydrophilic silicon dioxide having a specific surface area of 100 to 400 m²/g and a water affinity of not less than 75 %.

When the specific surface area is less than 50 m² /g, the suitable absorption rate which is an object of the present invention is not accomplished, and the initial absorbency under load (absorbency under load after 5 minutes) is reduced. Further, the elasticity after shearing of the gel is also not improved. On the other hand, no change is observed in performances of the absorbent composition even if the specific surface area exceeds 450 m²/g. The production cost of the hydrophilic silicon dioxide fine powder,however , increases and, therefore, it is not economical.

When the water affinity is less than 70 %, hydrophobic nature of the silicon dioxide becomes strong. Therefore, when the silicon dioxide is mixed into the water absorbing resin having a small content of fine powder as well as a narrow particle size distribution of the invention, the absorption rate of the resulting absorbent composition becomes low and the initial absorbency under load is also reduced. Furthermore, a hydrophobic silica has an inferior compatibility with the hydrophilic water absorbing resin and, therefore, it is not tightly adhered on the surface of the water absorbing resin (A), sometimes. When (B) is not tightly adhered on the surface of (A), dusting is liable to happen.

The silicon dioxide fine powder having a water affinity of less than 70 % is normally referred to as a hydrophobic silica which is obtained by further reacting a silanol group at the surface of the hydrophilic silicon dioxide fine powder in the present invention with e.g. monomethyltrichlorosilane, dimethyldichlorosilane or trimethylchlorosilane to introduce an alkyl group. It is not suitable for the present invention by the above mentioned reason. The hydrophobic silica can be used in combination in an amount at which the resulting water affinity is within the defined range of the present invention.

The term "specific surface area" used herein means a value measured by Brunauer, Emmett and Teller's method. The term "water affinity" means a value indicating the proportion of the silicon dioxide which is suspended colloidally in a mixed solution (water:methanol=70:30). The smaller the value, the stronger the hydrophobic nature.

The particle size of the silicon dioxide fine powder may be not particularly limited if the specific surface area and water affinity satisfy the range of the present invention. Normally, it is said that the silicon dioxide fine powder has an initial particle size of 5 to 50 nm; see Ullmann's Encyclopedia of Industrial Chemistry, 5^{th} Ed., Vol A23, 1993, pp. 635-638. However, enlarged particles by aggregation of initial particles are present in a normal state. The particle size is normally 0.07 to 1 *µ*m, preferably 0.1 to 0.8 *µ*m.

In the present invention, the mixing ratio of the water absorbing resin (A) to the hydrophilic silicon dioxide fine powder (B) is 100 parts by weight (A): 0.05 to 5 parts by weight (B), preferably 0.1 to 2 parts by weight. When the amount of (B) is less than 0.05 parts by weight, sufficient elasticity modulus after shearing of the gel can not be obtained. On the other hand, when the amount of (B) exceeds 5 parts by weight, the effect corresponding to the amount can not be obtained, and it is not economical. Further, fine powders (B) which are not tightly adhered on the particle surface of (A), cause dusting and frictional resistance is increased. Therefore, workability in case of treatment of the absorbent composition is reduced and constant amount-feeding property becomes inferior.

An apparatus for mixing (A) with (B) may be a usual powder mixing apparatus, for example, a conical blender, Nauta blender, V-type blender, air-blending type mixer, turbulizer, screw type line blender or static mixer.

There is a suggestion concerning a process for producing a water absorbing resin. The process is as follows: A crosslinking agent and water are absorbed into a water absorbing resin and an inorganic powder, and then the obtained mixture is heated with stirring for conducting of crosslinking reaction and removal of water (JP-A-60-163956). However, the purpose of using the inorganic powder in this suggestion is for improving a blocking tendency in case of absorption of water into the water absorbing resin. Accordingly, it is essentially different from the present invention wherein performances of the absorbent composition have been positively improved by blending a hydrophilic silicon dioxide fine powder (B) having a specific property into the water absorbing resin (A) having a specific crosslinked structure and a particle size distribution so as to be used suitably for the disposable diaper. In the present invention, no heating operation is required after (B) is mixed.

As to the particle size distribution, the amount of particles having a particle size of larger than 850 *µ*m is not more than 10 % by weight, and the amount of particles having a particle size of smaller than 150 *µ* m is not more than 10 % by weight. Preferably, the amount of particles having a particle size of larger than 850 *µ* m is not more than 5 % by weight, and the amount of particles having a particle size of smaller than 150 *µ*m is not more than 5 % by weight. More preferably, the amount of particles having a particle size of larger than 710 *µ*m is not more than 5 % by weight, and the amount of particles having a particle size of smaller than 150 *µ*m is not more than 5 % by weight. Most preferably, the amount of particles having a particle size of larger than 600 *µ*m is not more than 5 % by weight, and the amount of particles having a particle size of smaller than 150 *µ*m is not more than 5 % by weight. When the amount of particles having a particle size of larger than 850 *µ*m exceeds 10 % by weight, the absorption rate of the resulting absorbent composition becomes too low to cause leakage of urine, sometimes, in case of application for a disposable diaper. On the other hand, when the amount of particles having a particle size of smaller than 150 *µ* m exceeds 10 % by weight, gel-blocking is liable to occur in case of contacting with urine, and leakage of urine sometimes happensbecause the elasticity modulus after shearing is reduced. In addition, since fine powders cause dusting and aggregation is caused by moisture absorption and, further, resin particles are liable to escape from the fibrous material, the workability in case of application for the disposable diaper becomes inferior.

Accordingly, by setting the particle size distribution within a specific narrow range, no gel-blocking occurs when urine is absorbed, and a suitable absorption rate for diffusing urine through the disposable diaper can be obtained. As a result, a disposable diaper having good surface dryness and no leakage is realized.

The particle size of "850 *µ*m" corresponds to 18 mesh according to JIS (Japanese Industrial Standard), 710 *µ*m corresponds to 22 mesh, 600 *µ*m corresponds to 28 mesh and 150 *µ*m corresponds to 100 mesh, respectively.

The particle size distribution can be adjusted by a method which has been hitherto known. For example, a method of controlling the amount of particles having a particle size of larger than 850 *µ*m and particles having a particle size of smaller than 150 *µ*m by passing an absorbent composition through a sieve is convenient. In addition to this method, there can be used a method of granulating fine particles (if necessary, a binder may be used) to obtain the objective particle size distribution, or a method of optimizing pulverization conditions to obtain the objective particle size distribution without screening. When using the reverse phased suspension polymerization method, the objective particle size distribution can also be obtained by selecting kind and amount of a dispersion stabilizer and optimizing the structure of a reaction tank and the stirring conditions, as is known to those skilled in the art.

The particle size distribution can also be adjusted at the stage of (A) alone. In this case, some fine powders can be sometimes produced by a mechanical shear force according to the apparatus used for mixing (A) with (B). Therefore, it is preferred to set the amount of particles having a particle size of smaller than 150 *µ* m below the above range.

In the absorbent composition of the present invention, absorbency under load with respect to artificial urine (i.e. aqueous solution containing 0.8 % sodium chloride, 2 % urea, 0.08 % magnesium sulfate and 0.03 % calcium chloride) is 20 to 50 g/g, the elasticity modulus after shearing of artificial urine absorbing gel is not less than 4·10⁻³ N/mm² (40,000 dyne/cm²) and the absorption rate is 10 to 90 seconds. The "artificial urine" is to be understood as a solution similar in composition and chemical behaviour to natural urine. Preferably, the absorbency under load with respect to artificial urine is 25 to 50 g/g, the elasticity modulus after shearing of the artificial urine absorbing gel is not less than 4,5.10⁻³N/mm² (45,000 dyne/cm²) and the absorption rate is 15 to 75 seconds.

By using an absorbent composition which satisfies these physical properties, as described above, a disposable diaper having good dry feeling and no leakage of urine after use for a long time can be realized. Particularly, it is useful for a thin type disposable diaper wherein the ratio of the absorbent to the fibrous material is large (e.g. a weight ratio of the absorbent to the fibrous material exceeds 30:70).

In the present invention, as the fibrous material, for example, there can be used cellulose fibers, modified cellulose fibers, organic synthetic fibers and a mixture thereof.

Examples of the cellulose fiber include natural fibers such as fluff pulp and cellulose type chemical fibers such as viscose rayon and acetate rayon. Examples of the organic synthetic fiber include polypropylene fiber, polyethylene fiber, polyamide fiber, polyacrylonitrile fiber, polyester fiber, polyvinyl alcohol fiber and heat adherent bicomponent fiber (e.g. bicomponent fiber wherein at least one sort of the above fiber is formed into sheath & core type, eccentric core-sheath type or side-by-side type, bicomponent fiber wherein at least two sorts of the above fibers are blended and bicomponent fiber wherein the surface layer of the above fiber is modified).

Among them, preferred fibrous materials are cellulose type natural fiber, polypropylene fiber, polyethylene fiber, polyester fiber, heat adherent bicomponent fiber and a mixture thereof. More preferable fibrous materials are fluff pulp, heat adherent bicomponent fiber formed into sheath & core type, eccentric core-sheath type or side-by-side type and a mixture thereof because they are superior in shape retention after the disposable diaper absorbed urine.

The above organic synthetic fiber is usually subjected to a hydrophilization treatment using a hydrophilizing agent such as a surfactant to apply for the disposable diaper, and it is suitable for the present invention.

Length and thickness of the above fibrous material is not specifically limited, and those having a length of 1 to 200 mm and a thickness of 0.1 to 100 denier can be suitably used. The shape may be anyone which is fiber-shaped. and examples thereof include web, thin cylinder, cut split yarn, staple and filament shape.

The amount of the absorbent composition is usually 20 to 80 % by weight, preferably 30 to 70 % by weight, more preferably 35 to 65 % by weight based on the total weight of the fibrous material and the absorbent composition. When the amount of the absorbent composition is less than 20% by weight, a conventional water absorbing resin can be satisfactorily used and the effect of using the absorbent composition of the present invention is little. On the other hand, when the amount exceeds 80 % by weight, it becomes difficult to fix the absorbent composition to the fibrous material.

In the present invention, the using methods of the absorbent composition may be those which have hitherto been known, for example, a method of mixing with the fibrous material, and a method of sandwiching between two-layers of fibrous materials, a method of dispersing between layers of a laminated fibrous material having at least three layers.

Further, the absorbent composition of the present invention can be used in combination with a conventional water absorbing resin, or it can be separately used at a separate part.

Usually, an absorber wherein the absorbent composition has been applied in the fibrous material by the above method is further laminated with an absorbent paper or a fibrous material and a liquid permeable top sheet is provided on the top and a liquid non-permeable back sheet is provided on the bottom and, further, leg gathers, waist gathers and a fastening tape are provided to give a disposable diaper.

If necessary, a suitable amount of bulking agents and additives such as organic powders (e.g. pulp powder, cellulose derivative and natural polysaccharides), inorganic powders (e.g. zeolite, super micronized hydrophobic silica and activated carbon), antiseptic agents, disinfectants, coloring agents, perfumes and surfactants can be added in the absorbent composition of the present invention unless the object and effect of the present invention is damaged.

The following Examples and Comparative Examples further illustrate the present invention in detail. Absorbency under pressure-free state, absorbency under pressure and initial elasticity modulus of the absorbent composition, as well as elasticity modulus after shearing of the gel and absorption rate and performances of the disposable diaper were measured by the following method. In the Examples and Comparative Examples, "parts" and "%" are by weight unless otherwise stated.

### Absorbency under pressure-free state:

An absorbent composition (1 g) was charged into a tea bag made from 250-mesh nylon net, which was then dipped in excess artificial urine for 30 minutes and then taken out and left for water removal for 15 minutes. Then, the weight increase was measured as absorbency under pressure-free state.

### Absorbency under load:

In a cylindrical plastic tube (30 mm in inside diameter, 60 mm in height) with a 250-mesh nylon net affixed on the bottom surface, 0.1 g of an absorbent composition was charged and uniformly spread, on which a weight having 30 mm in outside diameter was placed so that a 20 g/cm² load was applied. A tube containing the absorbent composition was dipped in a Petri dish (vessel of 12 cm in diameter) containing 60 ml of artificial urine, and left standing with the nylon net side down for 30 minutes. The 10-fold value of the increase in weight after 30 minutes was designated as absorbency under load. The term "initial absorbency under load" mentioned above means the value measured after 5 minutes according to the same manner.

### Elasticity modulus after shearing of gel:

1 g of an absorbent composition was charged into a 50 ml glass bottle with a stopper, to which was added 40 ml of artificial urine to prepare a 40-fold absorbing gel and then the bottle was sealed with the stopper. This bottle was maintained in a constant temperature bath at 40 °C for 5 hours. Then, 0.1 g of the gel maintained for 5 hours was placed on the center of a supporting table of a Crep Meter (manufactured by Yamaden Co., Ltd.). By pulling an upper cylinder down, the gel was compressed to 0.3 mm in thickness. By pulling the cylinder up and down, compression was repeated 20 times and stress and sectional area of the compressed gel after 20 times were measured. The stress to an unit surface area after shearing was calculated by the following formula:
Elasticity modulus after shearing of gel 10⁻⁵N/100mm² (dyne/cm²) = (F × 980)/S wherein F is the stress (g) after compression at 20 times, 980 is the acceleration of gravity (cm/second²) and S is the sectional area (cm²) of gel after compression at 20 times. The resulting value was designated as elasticity modulus after shearing of the gel.

### Absorption rate:

Artificial urine (50 cc, 25 ± 2 °C in temperature) and a magnetic stirrer (8 mm in diameter, 30 mm in length) were charged into a 100 ml beaker and then stirred at 600 rpm. 2 g of an absorbent composition was introduced into a swirl. Immediately after introduction, the absorbent composition began to absorb artificial urine. As a result, the swirl disappeared and the liquid surface became horizontal. This period of time was measured as absorption rate.

### Evaluation of dryness using disposable diaper:

Preparation of model disposable diaper: On a polyethylene sheet cut into a rectangular piece of 14 cm × 36 cm in size, a tissue paper having the same size and a fluff pulp having a weight of 200 g/m² are laminated. Then, 7.5 g of a water absorbing resin is uniformly spreaded on the fluff pulp and, further, a fluff pulp having a weight of 100 g/m², a tissue paper and a non-woven fabric are laminated in this order. The resulting laminate was pressed at a pressure of 5 kg/cm² for 90 seconds to prepare a model disposable diaper.

Measurement of dryness: 80 ml of artificial urine (colored with blue ink) is poured into the center of the model diaper. 30 Minutes later, a shear force is applied on the surface of the diaper by reciprocating rolling 10 times using a roller having weight of 5 kg. Then, 80 ml of artificial urine is further poured into the center of the model disposable diaper and a shear force is applied again after 30 minutes, according to the same manner as that described above. This operation is repeated one more time. After the third operation, dryness of the disposable diaper surface was evaluated by 10 panellers according to the following four criteria:
- ⓞ :: Good dry feeling
- 0:: Slightly wet but satisfactory dry feeling
- Δ:: Poor dry feeling, wet state
- ×:: No dry feeling, completely wet state

Measurement of diffusion area: After the evaluation of dryness, the area where artificial urine is absorbed and spreaded (colored in blue) was measured as diffusion area.

### Production Example 1 of water absorbing resin particles

77 g of sodium acrylate, 23 g of acrylic acid, 0.4 g of N,N'-methylenebisacrylamide and 296 g of deionized water were charged into a 1 liter glass reaction vessel, and the temperature of the content was maintained at 5 °C with stirring and mixing. Nitrogen gas was introduced to reduce the dissolved oxygen content of the solution to 1 ppm or less. Then, 1 g of a 1 % aqueous solution of hydrogen peroxide, 1.2 g of a 0.2 % aqueous solution of ascorbic acid and 2.5 g of a 2% aqueous solution of 2,2'-azobisamidinopropane hydrochloride were added to initiate polymerization, and a hydrogel polymer (I) was obtained after polymerization for about 5 hours. 6 g of a 5 % aqueous solution of ethylene glycol diglycidyl ether was added to the hydrogel polymer while kneading with a kneader to knead uniformly. The hydrogel was subjected to hot-air drying at 130 to 150 °C and pulverized. Then, the particle size was adjusted such that the proportion of particles of 850 to 150 *µ*m (containing 4 % of particles having a particle size of larger than 850 *µ*m and 2 % of particles having a particle size of smaller than 150 *µ*m) becomes 94 % to obtain water absorbing resin particles (R-1).

### Production Example 2 of water absorbing resin particles

The hydrogel polymer (I) obtained in Production Example 1 was subjected to hot-air drying at 130 to 150 °C and pulverized. Then, the particle size was adjusted such that the proportion of particles of 850 to 150 *µ*m (containing 4 % of particles having a particle size of larger than 850 *µ*m and 2 % of particles having a particle size of smaller than 150 *µ*m) becomes 94 % to obtain crosslinked polymer particles (II). 3 g of a 10 % aqueous solution of ethylene glycol diglycidyl ether was sprayed uniformly to 100 g of the crosslinked polymer particles (II) with stirring at high speed, followed by heat treatment at about 140 °C for 30 minutes to obtain water absorbing resin particles (R-2). The particle size distribution of (R-2) was almost the same as that of (II).

### Production Example 3 of water absorbing resin particles

100 g of acrylic acid , 0.5 g of neopentyl glycol triallyl ether and 330 g of deionized water were charged into a 1 liter glass reaction vessel, and the temperature of the content was maintained at 5 °C with stirring and mixing. Nitrogen gas was introduced to reduce the dissolved oxygen content of the solution to 1 ppm or less . Then, 1 g of a 1 % aqueous solution of hydrogen peroxide, 1.2 g of a 0.2 % aqueous solution of ascorbic acid and 2.5 g of a 2% aqueous solution of 2,2'-azobisamidinopropane hydrochloride were added to initiate polymerization, and a hydrogel polymer was obtained after polymerization for about 5 hours. 116 g of a 35 % aqueous solution of sodium hydroxide was added to the hydrogel polymer while kneading with an extruder having a porous-disk plate to knead uniformly to obtain a hydrogel polymer (III) wherein 73 mol % of acrylic acid has been neutralized. 6 g of a 5 % aqueous solution of ethylene glycol diglycidyl ether was added to the hydrogel polymer (III), followed by uniform kneading. The hydrogel was dried with a drum drier the surface temperature of which is 180 °C and pulverized. Then, the particle size was adjusted such that the proportion of particles of 850 to 150 *µ*m (containing 2 % of particles having a particle size of larger than 850 *µ*m and 4 % of particles having a particle size of smaller than 150 *µ*m) becomes 94 % to obtain water absorbing resin particles (R-3).

### Production Example 4 of water absorbing resin particles

The hydrogel polymer (III) obtained in Production Example 3 was dried with a drum drier the surface temperature of which is 180 °C and pulverized. Then the particle size was adjusted such that the proportion of particles of 850 to 150 *µ*m (containing 2 % of particles having a particle size of larger than 850 *µ*m and 4 % of particles having a particle size of smaller than 150 *µ*m) becomes 94 % to obtain crosslinked polymer particles (IV). 4 g of a 10 % aqueous solution of polyamide polyamine epichlorohydrin resin was sprayed uniformly to 100 g of the crosslinked polymer particles (IV) with stirring at high speed, followed by heat treatment at about 140 °C for 30 minutes to obtain water absorbing resin particles (R-4). The particle size distribution of (R-4) was almost the same as that of (IV).

### Production Example 5 of water absorbing resin particles

65.4 g of acrylic acid and 78.6 g of deionized water were charged into a 200 ml flask, to which was added dropwise 56.0 g of a 48 % aqueous solution of sodium hydroxide under stirring with cooling to 20 to 30 °C to neutralize 74 mol % of acrylic acid. 0.15 g of N,N' -methylenebisacrylamide was dissolved in the resulting monomer solution, to which was added 0.1 g of potassium persulfate to dissolve at room temperature. Then, nitrogen gas was introduced to reduce the dissolved oxygen content of the solution to 1 ppm or less.

To a 1 liter flask equipped with a reflux condenser, 400 g of n-hexane was charged and 3 g of sorbitan monostearate was dissolved. Then, nitrogen gas was introduced to reduce the dissolved oxygen content in the solution to 1 ppm or less. Then, the temperature was maintained at 60 °C in a hot water bath and an aqueous monomer solution containing the above potassium peroxide was added dropwise with stirring to conduct polymerization for 3 hours, followed by maturing under reflux for 2 hours to obtain a dispersion of a pearly hydrogel polymer. Then, 3 g of a 1 % aqueous solution of ethylene glycol diglycidyl ether was added, followed by crosslinking under reflux for one hour and additional filtration of the hydrogel polymer from the polymer suspension. Further, the filtrated polymer was centrifuged and dehydrated to obtain a pearly hydrogel polymer. The hydrogel polymer was vacuum-dried at 90 to 95 °C and the particle size was adjusted such that the proportion of particles of 850 to 150 *µ*m (containing 1 % of particles having a particle size of larger than 850 *µ*m and 8 % of particles having a particle size of smaller than 150 *µ* m) becomes 91 % to obtain water absorbing resin particles (R-5).

### Production Example 6 of water absorbing resin particles

3 g of a 5 % aqueous solution of ethylene glycol diglycidyl ether was added to the hydrogel polymer (III) obtained in Production Example 3 to knead uniformly. The hydrogel was dried with a drum drier of which surface temperature is 180 °C and pulverized. Then, the particle size was adjusted such that the proportion of particles of 850 to 150 *µ*m (containing 8 % of particles having a particle size of larger than 850 *µ*m and 6 % of particles having a particle size of smaller than 150 *µ*m) becomes 86 % to obtain water absorbing resin particles (R-6).

### Comparative Production Examples 1 and 2 of water absorbing resin particles

Crosslinked polymer particles (II) obtained in Production Example 2 and crosslinked by only the crosslinking agent (a) were designated as comparative water absorbing resin particles (R-7), and crosslinked polymer particles (IV) obtained in Production Example 4 and crosslinked by only the crosslinking agent (a) were designated as comparative water absorbing resin particles (R-8).

### Comparative Production Example 3 of water absorbing resin particles

According to the same manner as that described in Production Example 3 except for adjusting the particle size such that the proportion of particles of 850 to 150 *µ*m (containing 15 % of particles having a particle size of larger than 850 *µ*m and 1 % of particles having a particle size of smaller than 150 *µ*m) becomes 84 %, comparative water absorbing resin particles (R-9) were obtained.

### Comparative Production Example 4 of water absorbing resin particles

According to the same manner as that described in Production Example 3 except for adjusting the particle size such that the proportion of particles of 850 to 150 *µ*m (containing 1 % of particles having a particle size of larger than 850 *µ*m and 15 % of particles having a particle size of smaller than 150 *µ*m) becomes 84 %, comparative water absorbing resin particles (R-10) were obtained.

### Examples 1 to 6

To water absorbing resin particles (R-1) to (R-6) (100 g each), 0.8 g of hydrophilic silicon dioxide fine powder having a specific surface area of 200 ± 20 m²/g and a water affinity of 100 % ("REOLOSIL QS-102", manufactured by Tokuyama Soda Co., Ltd.) was added and completely mixed by a V-type mixer to obtain an absorbent composition of the present invention. The particle size distribution was changed scarcely by adding the hydrophilic silicon dioxide fine powder. Performances of the resulting absorbent compositions were determined. The results are shown in Table 1.

### Examples 7 and 8

To 100 g of water absorbing resin particles (R-3), 0.8 g of different kinds of hydrophilic silicon dioxide fine powder was added and completely mixed by a V-type mixer to obtain an absorbent composition of the present invention. The particle size distribution was scarcely changed by adding the hydrophilic silicon dioxide fine powder. Performances of the resulting absorbent compositions were determined. The results are shown in Table 1.
(1) Hydrophilic silicon dioxide fine powder having a specific surface area of 380 ± 30 m²/g and a water affinity of 100 % ("AEROSIL 380", manufactured by Japan Aerosil Co., Ltd.)
(2) Hydrophilic silicon dioxide fine powder having a specific surface area of 140 ± 20 m²/g and a water affinity of 98 % ("REOLOSIL QS-10", manufactured by Tokuyama Soda Co., Ltd.)

### Examples 9 and 10

To 100 g of water absorbing resin particles (R-3), 0.3 g (Example 9) or 2 g (Example 10) of hydrophilic silicon dioxide fine powder "REOLOSIL QS-102"
was added and completely mixed by a V-type mixer to obtain an absorbent composition of the present invention. The particle size distribution was scarcely changed by adding the hydrophilic silicon dioxide fine powder. Performances of the resulting absorbent compositions were determined. The results are shown in Table 1.

### Production Example 7 of water absorbing resin particles

To the hydrogel polymer (I) obtained in Production Example 1, 1.5 g of a 5 % aqueous solution of ethylene glycol diglycidyl ether was added to knead uniformly. The hydrogel was subjected to hot-air drying at 130 to 150 °C and pulverized. Then, the particle size was adjusted such that the proportion of particles of 850 to 150 *µ*m (containing 1 % of particles having a particle size of larger than 850 *µ*m and 4 % of particles having a particle size of smaller than 150 *µ*m) becomes 95 % to obtain crosslinked polymer particles. 3 g of a 10 % aqueous solution of ethylene glycol diglycidyl ether was sprayed uniformly to 100 g of the crosslinked polymer particles with stirring at high speed, followed by heat treatment at about 140 °C for 30 minutes to obtain water absorbing resin particles (R-11).

### Production Example 8 of water absorbing resin particles

To the hydrogel polymer (III) obtained in Production Example 3, 1.5 g of a 5 % aqueous solution of ethylene glycol diglycidyl ether was added to knead uniformly. The hydrogel was subjected to hot-air drying at 130 to 150 °C and pulverized. Then, the particle size was adjusted such that the proportion of particles of 850 to 150 *µ*m (containing 1 % of particles having a particle size of larger than 850 *µ*m and 4 % of particles having a particle size of smaller than 150 *µ*m) becomes 95 % to obtain crosslinked water absorbing resin particles. 3 g of a 10 % aqueous solution of ethylene glycol diglycidyl ether was sprayed uniformly to 100 g of the crosslinked polymer particles with stirring at high speed, followed by heat treatment at about 140 °C for 30 minutes to obtain water absorbing resin particles (R-12).

### Production Example 9 of water absorbing resin particles

According to the same manner as that described in Production Example 8 except for adjusting the particle size to 710 to 150 *µ*m (containing 2 % of particles having a particle size of larger than 710 *µ*m and 4 % of particles having a particle size of smaller than 150 *µ*m), water absorbing resin particles (R-13) were obtained.

### Production Example 10 of water absorbing resin particles

According to the same manner as that described in Production Example 8 except for adjusting the particle size to 600 to 150 *µ*m (containing 2 % of particles having a particle size of larger than 600 *µ*m and 4 % of particles having a particle size of smaller than 150 *µ*m), water absorbing resin particles (R-14) were obtained.

### Examples 11 to 14

To water absorbing resin particles (R-11) to (R-14) (100 g each), 0.3 g of hydrophilic silicon dioxide fine powder having a specific surface area of 200 ± 20 m²/g and a water affinity of 100 % ("REOLOSIL QS-102", manufactured by Tokuyama Soda Co., Ltd.) was added and completely mixed by a V-type mixer to obtain an absorbent composition of the present invention. The particle size distribution was scarcely changed by adding the hydrophilic silicon dioxide fine powder. Performances of the resulting absorbent compositions were determined. The results are shown in Table 1.

### Comparative Examples 1 to 6

The measurement results of water absorbing resin particles (R-1) to (R-6) to which no hydrophilic silicon dioxide fine powder has been added are shown in Table 2.

### Comparative Examples 7 to 10

To comparative water absorbing resin particles (R-7) to (R-10) (100 g each), 0.8 g of "REOLOSIL QS-102" was added and completely mixed by a V-type mixer to obtain a comparative absorbent composition. The particle size distribution was scarcely changed by adding the hydrophilic silicon dioxide fine powder. Performances of the resulting absorbent compositions were determined. The results are shown in Table 2.

### Comparative Example 11

To 100 g of water absorbing resin particles (R-3), 0.03 g of "REOLOSIL QS-102" was added and completely mixed by a V-type mixer to obtain a comparative absorbent composition. The particle size distribution was scarcely changed by adding the hydrophilic silicon dioxide fine powder. Performances of the resulting absorbent compositions were determined. The results are shown in Table 2.

### Comparative Example 12

To 100 g of water absorbing resin particles (R-3), 0.8 g of "REOLOSIL MT-10"(manufactured by Tokuyama Soda Co., Ltd.) having a water affinity of 58% was added and completely mixed by a V-type mixer to obtain a comparative absorbent composition. The particle size distribution was scarcely changed by adding the hydrophilic silicon dioxide fine powder. Performances of the resulting absorbent compositions were determined. The results are shown in Table 2 below.

**Table 1**

| Example No. | Performance of absorbent composition of the present invention | | | | | Performance of disposable diaper | |
|---|---|---|---|---|---|---|---|
| | Absorbency under load (ml/g) | | Initial elasticity | Elasticity modulus | Absorption rate | Dryness | Diffusion area |
| | 5 min. value | 30 min. value | modulus (*1) | after shearing (*1) | (second) | | (cm²) |
| 1 | 22 | 29 | 10.4 | 8.4 | 45 | ⓞ | 208 |
| 2 | 28 | 37 | 8.3 | 6.9 | 39 | ○ | 213 |
| 3 | 26 | 34 | 11.2 | 9.8 | 33 | ⓞ | 238 |
| 4 | 31 | 42 | 8.6 | 7.2 | 30 | ⓞ - ○ | 225 |
| 5 | 24 | 30 | 9.2 | 7.5 | 48 | ⓞ | 216 |
| 6 | 21 | 28 | 8.5 | 6.7 | 42 | ○ | 202 |
| 7 | 27 | 34 | 11.3 | 9.8 | 32 | ⓞ | 236 |
| 8 | 25 | 34 | 11.0 | 9.7 | 35 | ⓞ | 227 |
| 9 | 24 | 33 | 10.2 | 8.5 | 42 | ⓞ - ○ | 204 |
| 10 | 27 | 34 | 11.5 | 10.1 | 28 | ⓞ | 244 |
| 11 | 26 | 33 | 9.6 | 8.8 | 36 | ⓞ | 230 |
| 12 | 30 | 40 | 10.2 | 9.5 | 27 | ⓞ | 242 |
| 13 | 32 | 42 | 10.1 | 9.5 | 25 | ⓞ | 245 |
| 14 | 35 | 44 | 10.1 | 9.4 | 22 | ⓞ | 243 |

| | | | | | | | |
|---|---|---|---|---|---|---|---|
| Note: *1 = 10⁻³N/mm² (10⁴dyne/cm²) | | | | | | | |

**Table 2**

| Comparative | Performance of absorbent composition of the present invention | | | | | Performance of disposable diaper | |
|---|---|---|---|---|---|---|---|
| Example No. | Absorbency under load (ml/g) | | Initial elasticity | Elasticity modulus | Absorption rate | Dryness | Diffusion area |
| | 5 min. value | 30 min. value | modulus (*1) | after shearing (*1) | (second) | | (cm²) |
| 1 | 13 | 27 | 8.7 | 5.6 | 98 | Δ | 168 |
| 2 | 17 | 34 | 6.8 | 4.4 | 94 | Δ - × | 164 |
| 3 | 15 | 30 | 9.1 | 5.8 | 105 | Δ | 176 |
| 4 | 21 | 36 | 7.2 | 4.8 | 96 | Δ - × | 178 |
| 5 | 13 | 28 | 8.5 | 5.5 | 108 | Δ | 172 |
| 6 | 9 | 24 | 7.4 | 4.0 | 116 | × | (*2) |
| 7 | 11 | 14 | 5.6 | 3.3 | 48 | × | (*2) |
| 8 | 10 | 18 | 5.8 | 3.5 | 45 | × | 167 |
| 9 | 8 | 28 | 10.8 | 9.5 | 143 | Δ - × | (*2) |
| 10 | 13 | 29 | 8.4 | 3.8 | 26 | × | (*2) |
| 11 | 16 | 30 | 9.2 | 5.8 | 98 | Δ | 175 |
| 12 | 9 | 25 | 10.5 | 9.5 | 124 | Δ - × | (*2) |

| | | | | | | | |
|---|---|---|---|---|---|---|---|
| Note: *1 = 10⁻³N/cm² (10⁴ dyne/cm²) | | | | | | | |
| *2 = Leakage was arisen | | | | | | | |

The absorbent composition of the present invention has the following advantageous effects.
(1) The absorbent composition has a high absorbency under load. Further, the absorbent composition is also superior in initial absorbency under load. Accordingly, in case of application for the disposable diaper, it absorbs urine sufficiently even in a state at which the user's weight is applied.
(2) The initial elasticity modulus is high and, therefore, in case of application for the disposable diaper, the absorbing gel is not deformed or broken due to the user's weight.
(3) The absorbent composition is superior in elasticity modulus after shearing with respect to the artificial urine absorbing gel. Accordingly, in case of application for the disposable diaper, it effects a stable absorption rate even if a shear force due to the user's locomotion (e.g. crawling, shifting of hip, turning-over, etc.) is applied, which results in repeatable absorption of urine.
(4) The absorbent composition has a suitable absorption rate and, therefore, urine can be widely diffused through the disposable diaper.
(5) The dry feeling of the gel after absorption of urine is excellent, which results in non-slippery feeling.
(6) The absorbent composition has a small content of fine powder and a narrow particle size distribution. Accordingly, no gel-blocking occurs when it absorbs urine in case of application for the disposable diaper and urine can be widely diffused through the disposable diaper.
(7) In case of application for the disposable diaper, no dusting occurs and the fluidity of the powder is excellent, which results in the improvement of workability.
(8) The absorbent composition does not escape from the fibrous material. Accordingly, productivity in the production of the disposable diaper is improved (production loss is reduced).
(9) The absorbent composition can be produced by a simple operation, i.e. mixing of the water absorbing resin (A) with hydrophilic silicon dioxide fine powder (B).

Further, the disposable diaper wherein the absorbent composition obtained by the process of the present invention is used in the fibrous material has the following characteristics.
(1) Dry feeling and non-slippery feeling of the surface of the disposable diaper after absorption of urine are excellent.
(2) Since the water absorbing resin has a suitable absorption rate, the diffusion property of urine through the disposable diaper is excellent, whereby urine can be sufficiently absorbed through the disposable diaper.
(3) There is no trouble of urine leakage.

With the above effects, the absorbent composition obtained by the process of the present invention is useful for disposable diapers (e.g. disposable diapers for infants or adults). Particularly, it is suitable for thin type disposable diapers wherein the ratio of the absorbent to the fibrous material is large.

Further, the absorbent composition can be used suitably for other water-absorptive pads and sanitary materials (e.g. sanitary napkin, incontinence pad, mother's milk pad, under pad for operation, pet sheet, etc.)

## Claims

1. A powdery or particulate absorbent composition comprising a mixture of 100 parts by weight of water absorbing resin particles (A) and 0.05 to 5 parts by weight of a hydrophilic silicon dioxide fine powder (B), said composition having a particle size distribution such that the amount of particles having a particle size of larger than 850 *µ*m is not more than 10 % by weight and the amount of particles having a particle size of smaller than 150 *µ*m is not more than 10 % by weight;
wherein said water absorbing resin particles (A) comprise an acrylic acid salt and/or an acrylic acid as a main monomer of the resulting polymer, said water absorbing resin particleshaving a structure which is crosslinked with a first crosslinking agent (a) having at least two double bonds capable of copolymerizing with the monomer and a second crosslinking agent (b) having at least two functional groups capable of covalently binding to a carboxylic group; and wherein
said hydrophilic silicon dioxide fine powder (B) has a specific surface area of 50 to 450 m²/g and a water affinity of not less than 70 %.

2. The absorbent composition according to claim 1, wherein said water absorbing resin particles (A) are obtainable by polymerizing a monomer mixture of an acrylic acid and an acrylic acid salt with a crosslinking agent (a) and reacting carboxylic groups in the resulting polymer with a crosslinking agent (b).

3. The absorbent composition according to claim 1, wherein said water absorbing resin particles (A) are obtainable by polymerizing an acrylic acid with a crosslinking agent (a) and partially neutralizing carboxylic groups in the resulting polymer with an alkali metal salt, and further crosslinking carboxylic groups in the polymer with a crosslinking agent (b).

4. The absorbent composition according to claim 1, wherein said water absorbing resin particles are obtainable by further crosslinking the vicinity of the surface of the water absorbing resin particles obtained in claims 2 or 3 with a crosslinking agent (b).

5. The absorbent composition according to any one of claims 1 to 4, wherein the amount of said crosslinking agent (a) is 0.05 to 5 weight % based on the weight of said monomer, and the amount of said crosslinking agent (b) is 0.01 to 3 % by weight based on the weight of said monomer.

6. The absorbent composition according to any one of claims 1 to 5, wherein the weight ratio of said water absorbing resin particles (A) to said hydrophilic silicon dioxide fine powder (B) is 100 parts by weight (A): 0.1 to 2 parts by weight (B).

7. The absorbent composition according to any one of claims 1 to 6, wherein the amount of said particles having a particle diameter of larger than 850 *µ*m is not more than 5 % by weight, and the amount of said particles having a particle diameter of smaller than 150 *µ*m is not more than 5 % by weight.

8. The absorbent composition according to any one of claims 1 to 7, wherein said composition has a particle size distribution such that the amount of said particles having a particle diameter of larger than 710 *µ*m is not more than 5 % by weight, and the amount of said particles having a particle diameter of smaller than 150 *µ*m is not more than 5 % by weight.

9. The absorbent composition according to any one of claims 1 to 8, wherein said composition has a particle size distribution such that the amount of said particle having a particle diameter of larger than 600 *µ*m is not more than 5 % by weight, and the amount of said particles having a particle diameter of smaller than 150 *µ*m is not more than 5 % by weight.

10. The absorbent composition according to any one of claims 1 to 9, wherein the absorbency under load with respect to artificial urine is 20 to 50 g/g, the elasticity modulus after shearing of the artificial urine absorbing gel is not less than 4·10⁻³N/mm² (40,000 dyne/cm²) and the absorption rate is 10 to 90 seconds.

11. The use of the absorbent composition according to any one of claims 1 to 10 in a disposable diaper.

12. A disposable diaper comprising the absorbent composition according to any one of claims 1 to 10 and a fibrous material.

13. The disposable diaper according to claim 12, wherein said fibrous material is at least one material selected from the group consisting of fluff pulp, heat adherent bicomponent fiber and a mixture thereof.

14. The disposable diaper according to any one of claims 12 and 13, wherein the amount of said absorbent composition for disposable diaper is 30 to 70 % by weight based on the total weight of said fibrous material and said composition.

## Patentansprüche

1. Pulverförmige oder aus Teilchen bestehende absorbierende Zusammensetzung umfassend ein Gemisch aus 100 Gewichtsteilen wasserabsorbierender Harzteilchen (A) und 0,05 bis 5 Gewichtsteilen hydrophilen, feinkörnigen Siliciumdioxidpulvers (B), wobei die Zusammensetzung eine derartige Teilchengrößenverteilung aufweist, daß die Menge an Teilchen mit einer Teilchengröße von größer als 850 µm höchstens 10 Gew.-% ist und daß die Menge an Teilchen mit einer Teilchengröße von kleiner als 150 µm höchstens 10 Gew.-% ist;
wobei die wasserabsorbierenden Harzteilchen (A) ein Acrylsäuresalz und/oder eine Acrylsäure als ein Hauptmonomer des erhaltenen Polymers umfassen, wobei die wasserabsorbierenden Harzteilchen eine Struktur aufweisen, die mit einem ersten Vernetzungsmittel (a), das mindestens zwei Doppelbindungen aufweist, die mit dem Monomer copolymerisieren können, und einem zweiten Vernetzungsmittel (b), das mindestens zwei funktionelle Gruppen aufweist, die an eine Carbonsäuregruppe kovalent binden können, vernetzt ist; und wobei das hydrophile, feinkörnige Siliciumdioxidpulver (B) eine spezifische Oberfläche von 50 bis 450 m²/g und eine Wasseraffinität von mindestens 70% aufweist.

2. Absorbierende Zusammensetzung nach Anspruch 1, wobei die wasserabsorbierenden Harzteilchen (A) durch Polymerisieren eines Monomerengemisches aus einer Acrylsäure und einem Acrylsäuresalz mit einem Vernetzungsmittel (a) und Umsetzen von Carbonsäuregruppen in dem so erhaltenen Polymer mit einem Vernetzungsmittel (b) erhältlich sind.

3. Absorbierende Zusammensetzung nach Anspruch 1, wobei die wasserabsorbierenden Harzteilchen (A) durch Polymerisieren einer Acrylsäure mit einem Vernetzungsmittel (a), teilweises Neutralisieren von Carbonsäuregruppen in dem erhaltenen Polymer mit einem Alkalimetallsalz und dann Vernetzen von Carbonsäuregruppen in dem Polymer mit einem Vernetzungsmittel (b) erhältlich sind.

4. Absorbierende Zusammensetzung nach Anspruch 1, wobei die wasserabsorbierenden Harzteilchen durch weiteres Vernetzen der Umgebung der Oberfläche der in den Ansprüchen 2 oder 3 erhaltenen wasserabsorbierenden Harzteilchen mit einem Vernetzungsmittel (b) erhältlich sind.

5. Absorbierende Zusammensetzung nach einem der Ansprüche 1 bis 4, wobei die Menge des Vernetzungsmittels (a) 0,05 bis 5 Gew.-% auf der Basis des Gewichts des Monomers ist, und die Menge des Vernetzungsmittels (b) 0,01 bis 3 Gew.-% auf der Basis des Gewichts des Monomers ist.

6. Absorbierende Zusammensetzung nach einem der Ansprüche 1 bis 5, wobei das Gewichtsverhältnis der wasserabsorbierenden Harzteilchen (A) zu dem hydrophilen, feinkörnigen Siliciumdioxidpulver (B) 100 Gewichtsteile (A) : 0,1 bis 2 Gewichtsteile (B) ist.

7. Absorbierende Zusammensetzung nach einem der Ansprüche 1 bis 6, wobei die Menge der Teilchen mit einem Teilchendurchmesser von größer als 850 µm höchstens 5 Gew.-% und die Menge der Teilchen mit einem Teilchendurchmesser von kleiner als 150 µm höchstens 5 Gew.-% beträgt.

8. Absorbierende Zusammensetzung nach einem der Ansprüche 1 bis 7, wobei die Zusammensetzung eine derartige Teilchengrößenverteilung aufweist, daß die Menge der Teilchen mit einem Teilchendurchmesser von größer als 710 µm höchstens 5 Gew.-% und die Menge der Teilchen mit einem Teilchendurchmesser von kleiner als 150 µm höchstens 5 Gew.-% beträgt.

9. Absorbierende Zusammensetzung nach einem der Ansprüche 1 bis 8, wobei die Zusammensetzung eine derartige Teilchengrößenverteilung aufweist, daß die Menge der Teilchen mit einem Teilchendurchmesser von größer als 600 µm höchstens 5 Gew.-% und die Menge der Teilchen mit einem Teilchendurchmesser von kleiner als 150 µm höchstens 5 Gew.-% beträgt.

10. Absorbierende Zusammensetzung nach einem der Ansprüche 1 bis 9, wobei das Absorptionsvermögen in Bezug auf künstlichen Urin 20 bis 50 g/g, das Elastizitätsmodul nach Scheren des künstlichen urinabsorbierenden Gels mindestens 4·10⁻³ N/mm² (40 000 Dyn/cm²) und die Saugfähigkeit 10 bis 90 Sekunden ist.

11. Verwendung der absorbierenden Zusammensetzung nach einem der Ansprüche 1 bis 10 in einer Wegwerfwindel.

12. Wegwerfwindel, umfassend die absorbierende Zusammensetzung nach einem der Ansprüche 1 bis 10 und ein faserförmiges Material.

13. Wegwerfwindel nach Anspruch 12, wobei das faserförmige Material mindestens ein Material, ausgewählt aus F[auschzellstoff, wärmehaftbeständiger Bikomponentenfaser und einem Gemisch davon ist.

14. Wegwerfwindel nach einem der Ansprüche 12 und 13, wobei die Menge der absorbierenden Zusammensetzung für Wegwerfwindeln 30 bis 70 Gew.-%, basierend auf der Gesamtmenge des faserförmigen Materials und der Zusammensetzung, beträgt.

## Revendications

1. Composition absorbante sous forme d'une poudre ou de particules comprenant un mélange de 100 parties en masse de particules de résine absorbant l'eau (A) et de 0,05 à 5 parties en masse d'une fine poudre de dioxyde de silicium hydrophile (B), ladite composition présentant une répartition de la taille particulaire telle que la quantité de particules ayant une taille particulaire supérieure à 850 µm n'est pas supérieure à 10% en masse et que la quantité de particules ayant une taille particulaire inférieure à 150 µm n'est pas supérieure à 10% en masse;
dans laquelle lesdites particules de résine absorbant l'eau (A) comprennent un sel d'un acide acrylique et/ou un acide acrylique en tant que monomère principal du polymère résultant, lesdites particules de résine absorbant l'eau ayant une structure qui est réticulée avec un premier agent de réticulation (a) comportant au moins deux doubles liaisons capables de se copolymériser avec le monomère et un second agent de réticulation (b) comportant au moins deux groupes fonctionnels capables de se lier d'une manière covalente à un groupe acide carboxylique; et dans laquelle
ladite fine poudre de dioxyde de silicium hydrophile (B) possède une surface spécifique comprise entre 50 et 450 m²/g et une affinité pour l'eau non inférieure à 70%.

2. Composition absorbante selon la revendication 1, dans laquelle lesdites particules de résine absorbant l'eau (A) peuvent être obtenues par polymérisation d'un mélange de monomères constitué d'un acide acrylique et d'un sel d'un acide acrylique avec un agent de réticulation (a) et par réaction des groupes carboxyliques dans le polymère résultant avec un agent de réticulation (b).

3. Composition absorbante selon la revendication 1, dans laquelle lesdites particules de résine absorbant l'eau (A) peuvent être obtenues par polymérisation d'un acide acrylique avec un agent de réticulation (a) et par neutralisation partielle des groupes carboxyliques dans le polymère résultant avec un sel d'un métal alcalin et ensuite par réticulation des groupes carboxyliques dans le polymère avec un agent de réticulation (b).

4. Composition absorbante selon la revendication 1, dans laquelle lesdites particules de résine absorbant l'eau peuvent être obtenues en réticulant de plus la zone proche de la surface des particules de résine absorbant l'eau obtenues dans les revendications 2 ou 3 avec un agent de réticulation (b).

5. Composition absorbante selon l'une quelconque des revendications 1 à 4, dans laquelle la quantité dudit agent de réticulation (a) est comprise entre 0,05 et 5% en masse sur la base de la masse dudit monomère et dans laquelle la quantité dudit agent de réticulation (b) est comprise entre 0,01 et 3% en masse sur la base de la masse dudit monomère.

6. Composition absorbante selon l'une quelconque des revendications 1 à 5, dans laquelle le rapport en masse entre lesdites particules de résine absorbant l'eau (A) et ladite fine poudre de dioxyde de silicium hydrophile (B) est égal à 100 parties en masse de (A) : 0,1 à 2 parties en masse de (B).

7. Composition absorbante selon l'une quelconque des revendications 1 à 6, dans laquelle la quantité desdites particules ayant un diamètre particulaire supérieur à 850 µm n'est pas supérieure à 5% en masse et la quantité desdites particules ayant un diamètre particulaire inférieur à 150 µm n'est pas supérieure à 5% en masse.

8. Composition absorbante selon l'une quelconque des revendications 1 à 7, dans laquelle ladite composition présente une répartition de la taille particulaire telle que la quantité desdites particules ayant un diamètre particulaire supérieur à 710 µm n'est pas supérieure à 5% en masse et que la quantité desdites particules ayant un diamètre particulaire inférieur à 150 µm n'est pas supérieure à 5% en masse.

9. Composition absorbante selon l'une quelconque des revendications 1 à 8, dans laquelle ladite composition présente une répartition de la taille particulaire telle que la quantité desdites particules ayant un diamètre particulaire supérieur à 600 µm n'est pas supérieure à 5% en masse et que la quantité desdites particules ayant un diamètre particulaire inférieur à 150 µm n'est pas supérieure à 5% en masse.

10. Composition absorbante selon l'une quelconque des revendications 1 à 9, dans laquelle la capacité d'absorption sous charge par rapport à l'urine artificielle est comprise entre 20 et 50 g/g, le module élastique après cisaillement du gel absorbant l'urine artificielle n'est pas inférieur à 4·10⁻³ N/mm² (40 000 dyne/cm²) et la vitesse d'absorption est comprise entre 10 et 90 secondes.

11. Utilisation de la composition absorbante selon l'une quelconque des revendications 1 à 10 dans une couche jetable.

12. Couche à jeter comprenant la composition-absorbante selon l'une quelconque des revendications 1 à 10 et un matériau fibreux.

13. Couche à jeter selon la revendication 12, dans laquelle ledit matériau fibreux est au moins un matériau choisi dans le groupe constitué par la pâte défibrée, une fibre à deux composants thermoadhésive et un de leurs mélanges.

14. Couche à jeter selon l'une quelconque des revendications 12 et 13, dans laquelle la quantité de ladite composition absorbante pour une couche à jeter est comprise entre 30 et 70% en masse sur la base de la masse totale dudit matériau fibreux et de ladite composition.
